# EUROPEAN PATENT SPECIFICATION

(11) **EP 0 502 987 B1**
(45) Date of publication and mention of the grant of the patent: **31.08.1994**
(21) Application number: 91901182.5
(22) Date of filing: 10.12.1990
(51) Int. Cl.: C07K 7/18, A61K 37/42

(54) **ACYLATED BRADYKININ ANTAGONISTS AND USES THEREFOR**
ACYLIERTE BRADYKININANTAGONISTEN UND DEREN VERWENDUNG
ANTAGONISTES ACYLES DE BRADYKININE ET LEURS UTILISATIONS

(30) Priority: 08.12.1989 US 447713
(43) Date of publication of application: 16.09.1992
(73) Proprietor: TRUSTEES OF BOSTON UNIVERSITY, Boston, Massachusetts 02215 (US)
(72) Inventor: GAVRAS, Haralambos, Marblehead, MA 01945 (US); LAMMEK, Bernard, 80-276 Gdansk (PL)
(74) Representative: Holdcroft, James Gerald, Dr.
(86) International application number: US9007268
(87) International publication number: WO9109055

(56) References cited:
- EP-A- 0 334 244
- EP-A- 0 370 453
- WO-A-86/07263
- WO-A-89/01781
- WO-A-89/09231
- Chemical Abstracts, vol. 95, no. 5, August 1981, (Columbus, Ohio, US), Sugavara, S., p. 114, abstract 35993q
- Chemical Abstracts, vol. 101, no. 3, 16 July 1984, (Columbus, Ohio, US), Vavrek, R.J. et al., p. 85, abstract 17550f
- Chemical Abstracts, vol. 111, no. 5, 31 July 1989, (Columbus, Ohio, US), Vavrek, R.J. et al., p. 81, abstract 33769w

## Description

### Background

Under normal conditions, bradykinin (BK) has a number of actions, some beneficial, some detrimental. Beneficial effects include the indirect vasodepressor action (mediated via stimulation of locally released and acting vasodilatory substances, such as EDRF and prostaglandins E₂ and I₂) and enhanced sensitivity to insulin-dependent glucose uptake and metabolism. Detrimental effects include nociceptive action on sensory nerve terminals (e.g. , pain, such as in burn blisters, neuralgia or tissue ischemia), broncho-constriction and airway inflammation (allergic asthma, rhinitis) , stimulation of sympathetic nerve terminals (pressor and arrhythmogenic effect in myocardial ischemia, especially when coronary arteries are atherosclerotic) , circulatory collapse in endotoxic shock or orthostatic hypotension (e.g., septicemia or diabetes with autonomic dysfunction).

All of the clinically relevant effects of bradykinin appear to be mediated via the classical B₂ receptors and, wherever tested, were found to be reversible by B₂ receptor antagonists. B₂ antagonists are very important pharmacologic tools in the research of mechanisms of hypertension, myocardial ischemia, post-myocardial infarction (MI) arrhythmia and congestive heart failure. They may also have a therapeutic potential in treating pain from burns, anginal pain and myocardial reperfusion arrhythmias, bronchial asthma and allergic rhinitis, toxic shock and circulatory collapse, orthostatic hypotension and possibly other B₂ mediated effects of bradykinin that are still ill-defined, such as pain, bowel hypermobility and diarrhea of chronic inflammatory intestinal disease, painful arthritis, neuralgia, etc.

The mechanism of bradykinin's actions and its interactions with other vasoactive systems has been investigated by various means. In one approach, the effect of bradykinin was studied by blocking its action via antibodies. Carretero et al., Hypertension, 3:18-22 (1981). Another approach made possible recently is the use of bradykinin analogs with B₂ binding properties. Using this approach, it was found that some bradykinin analogs are effective inhibitors of bradykinin. These analogs generally have modifications or additions to one or more of the nine bradykinin amino acids.

Two major classes of bradykinin receptors, B₁ and B₂, have been defined. In general, compounds which are B₂ antagonists are non-selective, and will act on both B₁ and B₂ receptors. The B₁ receptors are sensitive to the bradykinin metabolite des-Arg⁹-BK, and B₁-mediated effects are blocked by the specific antagonist des-Arg⁹-Leu⁸-BK. Effects mediated by B₂ receptors cannot be blocked by this type of antagonist. The key modification that confers B₂- antagonistic properties to an analog is replacement of the proline (Pro) residue at position 7 of bradykinin with a D-phenylalanine (D-Phe) residue. Other substitutions, such as replacement of proline by hydroxyproline (Hyp) in positions 2 or 3; substitution of the Phe residues in positions 5 and 8 with β-2-thienyl-L-alanine (Thi); or addition of one or two amino acid residues to the N-terminus, can potentiate the antagonistic properties of analogs. Stewart and Vavrek, In: Kinins IV, Greenbaum and Margolius (eds.), Plenum Publishing Co. New York, NY, pp. 537-542 (1986); Gavras and Gavras, Kidney International, 34: (supp. 26): S-60-S-62 (1988). Various bradykinin derivatives appear to exhibit different tissue specificity.

Antagonists of bradykinin have been used to study various cardiovascular effects that bradykinin exerts, either directly or via interaction with other vasoactive substances, by assessing the effects of chronic B₂ receptor inhibition of bradykinin on systemic or regional hemodynamics, cardiac function and other vasoactive systems. WO 86/07263 describes non-acylated bradykinin analogs having antagonist activity. One of the commonly used and most potent antagonists of bradykinin has the sequence: D-Arg-Arg-Pro-Hyp-Gly-Thi-Ser-D-Phe-Thi-Arg (D-Arg⁰,Hyp³,Thi^{5,8},D-Phe⁷-BK). This analog combines the chemical modifications that confer bradykinin antagonistic capacity at the B₂ receptors and a number of other desirable properties, such as high selectivity for vascular tissues and a relative resistance to enzymatic degradation which prolongs its plasma half-life to several minutes. The plasma half-life of bradykinin itself is only a few seconds. However, this analog his relatively low potency, which necessitates the use of high concentrations. Acylated derivatives of bradykinin are described in WO 89/01781. These analogs do not show an improved activity. Thus, more effective and efficient bradykinin inhibitors would be useful for studying the physiological effects of bradykinin and for diagnostic and therapeutic applications in bradykinin-mediated vascular disorders.

### Summary of the Invention

The present invention relates to a new class of bradykinin (BK) antagonists, which are acylated bradykinin analogs selected from 
O
X-C-Arg-Pro-Hyp-Gly-Thi-Ser-D-Phe-Thi-Arg,
O
X-C-D-Arg-Arg-Hyp-Hyp-Gly-Thi-Ser-D-Phe-Thi-Arg,
O
X-C-Lys-Lys-Arg-Pro-Pro-Gly-Thi-Ser-D-Phe-Thi-Arg and
O
X-C-D-Arg-Arg-Pro-Hyp-Gly-Thi-Ser-D-Phe-Thi-Arg,
wherein X is an organic substituent selected from the group consisting of: saturated cyclic compounds, unsaturated cyclic compounds, cyclic aromatic compounds, saturated heterocyclic compounds, unsaturated heterocyclic compounds and aromatic heterocyclic compounds.

The invention also relates to therapeutic methods of using the acylated bradykinin analogs. The new bradykinin antagonists of the invention exhibit much higher potency than presently-available bradykinin antagonists.

In one embodiment of the present invention, acylation of the bradykinin analog D-Arg-Arg-Pro-Hyp-Gly-Thi-Ser-D-Phe-Thi-Arg with adamantane acetic acid resulted in production of an acylated bradykinin analog having the following structure: 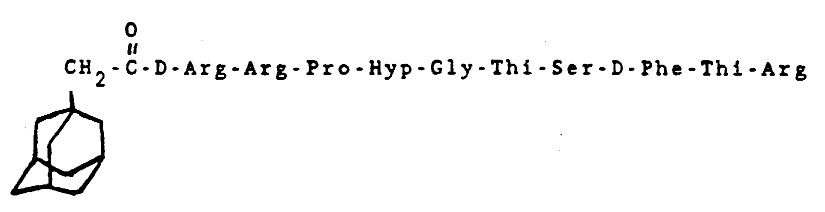
O
‖
CH2-C-D-Arg-Arg-Pro-Hyp-Gly-Thi-Ser-D-Phe-Thi-Arg
This acylated bradykinin analog has greatly enhanced antagonist potency and efficiency. That is, the resulting acylated bradykinin inhibitor has been shown to be ten times more potent than the base analog, D-Arg-Arg-Pro-Hyp-Gly-Thi-Ser-D-Phe-Thi-Arg.

A method of producing acylated bradykinin analogs having enhanced bradykinin antagonistic activity is also the subject of the present invention. In this method, a bradykinin analog having some bradykinin antagonistic activity is acylated at the N-terminal amino group of the peptide by reaction with a carboxylic acid, or derivative thereof. Acylated bradykinin analogs produced by the method have enhanced bradykinin-antagonist activity.

The acylated bradykinin analogs and modified acylated bradykinin analogs are useful therapeutic compounds which can be administered in any treatment context in which presently-available bradykinin analogs or other bradykinin inhibitors are tested. They are also useful in understanding the structure of bradykinin antagonists, as well as in designing even more potent and long-lasting antagonists.

Vasodepressor response in an individual may be reduced or inhibited using the present acylated bradykinin analogs of the present invention. An amount of the above-described acylated bradykinin analog sufficient to reduce or inhibit the vasodepressor response can be administered to an individual in need of treatment.
a Vascular disorders may be reduced, ameliorated or eliminated in an individual using the analogs of the present invention. Vascular disorders which can be treated include post-myocardial infarction arrythmia, angina, sympathetic discharge, cardiac arrythmia, diabetic neuropathy and ischemic heart disease.

The present acylated bradykinin analogs can be useful to treat many other B₂-mediated disorders related to bradykinin. For example, the present bradykinin antagonists can be used to treat bronchoconstriction, asthma, allergic rhinitis, endotoxin shock, inflammatory bowel disease and diarrhea. The analogs can be used as an anti-inflammatory agent in treating colds or flu, or as a cough suppressant. The analogs can also be used to treat pain which is related to excitation of central sensory neurons (e.g., burns, oro-facial pain, trigeminal neuralgia, migraine). Other forms of pain, such as pain related to the inflammatory response, increased vascular permeability and inflammatory edema, can also be relieved using the present method.

### Brief Description of the Figures

Figure 1 is a graph illustrating the level of bradykinin-inhibiting activity of an acylated bradykinin analog (new analog) compared to its non-acylated counterpart (model analog).

Figure 2 is a graph illustrating the recovery time after inhibition of bradykinin by an acylated bradykinin analog (new analog) and its non-acylated counterpart (model analog).

### Detailed Description of the Invention

A new class of bradykinin antagonists, generally referred to as acylated bradykinin analogs, has been synthesized by reaction of a bradykinin analog with a carboxylic compound, or derivative thereof, to accomplish acylation at the peptide N-terminus. Derivatives of carboxylic acids include, for example, chloride (COCl) and esters (COOR). These analogs are considerably more potent (i.e., have greater anatagonistic activity) than presently-available bradykinin analogs. In addition, acylated bradykinin analogs in which the bradykinin nonapeptide has been modified by substitution, addition or deletion of one or more amino acids in the peptide chain can be produced in order to provide a modified acylated bradykinin analog which, in addition to enhanced potency, has greater stability or resistance to destruction by cellular enzymes and is taken up more readily by cells. Enhanced potency or activity means that the acylated bradykinin analog has a greater antagonistic effect at the same dosage level, or the same antagonistic effect at a lower dosage level, compared to its non-acylated counterpart.

Although designed and synthesized by Stewart and Vavrek, the bradykinin analog having the sequence: D-Arg-Arg-Pro-Hyp-Gly-Thi-Ser-D-Phe-Thi-Arg, is referred to as the Stewart analog and is considered to be the most powerful bradykinin antagonist, both in vitro and in vivo. The key modification to convert bradykinin (which has the sequence: Arg-Pro-Pro-Gly-Phe-Ser-Pro-Phe-Arg) into a specific antagonist was the replacement of the Pro residue at position 7 with a D-Phe residue. Vavrek and Stewart, Peptides, 6:161-164 (1985).

Carboxylic compounds are characterized in that they have at least one carboxyl group (-COOH). Carboxylic derivatives, such as chloride salts (COCl) or esters (COOR) can also be used. Carboxyl groups or derivatives thereof, react with the N-terminal amino group of the bradykinin peptide to form an acylated bradykinin analog having enhanced antagonistic potency and efficiency. In addition to the carboxyl group, the organic substituent can have other functional groups, including, interalia, basic (NHR, N-R₁R₂, NH₂, NH₃) or acidic groups (e.g., SO₃H, HPO₄, COOH), halogen atoms (Cl, F. Br, I), or ester, ether, hydroxy, guanidine and thioester groups. Synthetic bradykinin analogs can be used as base peptides to produce the present acylated analogs. For example, the following sequences can be used: Arg-Pro-Hyp-Gly-Thi-Ser-D-Phe-Thi-Arg, D-Arg-Arg-Hyp-Hyp-Gly-Thi-Ser-D-Phe-Thi-Arg, Lys-Lys-Arg-Pro-Gly-Thi-Ser-D-Phe-Thi-Arg, Arg-Pro-Pro-Gly-Thi-Ser-D-Phe-Thi-Arg, and D-Arg-Arg-Pro-Hyp-Gly-Thi-Ser-D-Phe-Thi-Arg. See, Vavrek and Stewart, Peptides, 6:161-164 (1985); by Schachter et al., J. Pharmacol., 92:851-853 (1987) and Farmer et al., J.Pharmacol. and Exp. Ther., 248 (2):677-681 (1989), the teachings of which are incorporated herein by reference.

The acyl group: 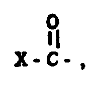
results from the reaction of a carboxylic acid, or derivative thereof, with the N-terminal amino group of the peptide. X can be a cyclic substituent (saturated, unsaturated or aromatic) or heterocyclic substituent (saturated, unsaturated or aromatic).

Carboxylic compounds which are useful include any carboxyl-containing chemical compound or derivative thereof which forms a stable acylated compound with the peptide of choice. Carboxylic compounds which are particularly useful include acetic acid, propionic acid and adamantaneacetic acid.

The peptides which serve as the base bradykinin analog can be made by known peptide synthesis techniques (e.g., solid phase peptide synthesis, solution synthesis, recombinant DNA techniques, or a combination of methods) or can be purchased, if commercially available. Synthesis of the base bradykinin analog by solid phase peptide synthesis is described in Example 1. The acylated bradykinin analogs of the present invention are made by acylating the N-terminal amino group of the peptide with a carboxylic compound, or derivative thereof, or by acylating the amino group of a selected amino acid and attaching it to the N-terminus of the peptide during peptide synthesis.

It has been shown (See Example 2) that the acylated bradykinin analogs of the present invention are potent bradykinin antagonists in a mammal. The method by which the antagonistic potency of the analogs was assessed and compared with that of the Stewart analog is described in Example 2. Results showed, for example, that the analog formed by acylating the Stewart bradykinin analog with adamantane acetic acid has greater antagonist potency (up to 10 times more potent) than the original (i.e. , non-acylated) peptide.

A method of producing an acylated bradykinin antagonist having enhanced potency is also the subject of the present invention. In this method, a bradykinin analog having bradykinin antagonistic activity is acylated at the N-terminal amino acid by reaction with a carboxylic compound or derivative thereof. The acylated bradykinin analog produced by the method has enhanced bradykinin antagonistic activity compared to the base analog.

Modified acylated bradykinin analogs are also the subject of the present invention. Modified acylated bradykinin analogs differ from the acylated bradykinin analog of the present invention in that the amino acid sequence of the bradykinin nonapeptide has been modified by the addition, substitution or deletion of one or more amino acids. These modified acylated bradykinin analogs have bradykinin-antagonistic properties, particularly for the B₂-receptors of bradykinin.

The acylated bradykinin analogs of the present invention are useful in studying the vasoactive properties of bradykinin and in investigating the activity of bradykinin in regulating cardiovascular function. The present acylated analogs can be used therapeutically to control vascular responses. The acylated analogs can be used as a pharmacological tool in the research of the mechanisms of post-myocardial infarction arrythmia, congestive heart failure, hypertension, angina, sympathetic discharge, cardiac arrythmia, diabetic neuropathy and ischemic heart disease.

The effects of bradykinin in an individual, which results in reduction or inhibition of the effects, can be antagonized using the compounds of the present invention. In particular, vasodepressor response can be reduced or inhibited in an individual using the present acylated bradykinin analogs. An amount of at least one acylated bradykinin analog sufficient to inhibit the vasodepressor response can be administered to an individual in whom treatment is desired. One or more acylated bradykinin analogs can be administered. The amount of the analog which is administered will vary depending upon the conditions being treated, the type and severity of the symptoms, the size of the individual being treated and the results sought. For example, a dosage of from about 3 mg to about 15 mg per day can be administered to obtain the desired results.

The acylated bradykinin analog is useful in the treatment of many other bradykinin-mediated disorders. For example, they can be used to treat bronchoconstriction, asthma, allergic rhinitis, endotoxin shock, inflammatory bowel disease and diarrhea. The present compositions can be used to treat pain, such as pain from burns, oro-facial pain, trigeminal neuralgia, and migraine which is related to excitation of central sensory neurons. Other forms of pain, such as pain related to inflammation, increased vascular permeability and inflammatory edema can also be relieved by administering the present acylated bradykinin antagonists to an afflicted individual.

The present invention will now be illustrated by the following Examples.

### EXAMPLE 1

### Synthesis of an Acylated Peptide Analog of Bradykinin

An acylated peptide analog of bradykinin having the structure: 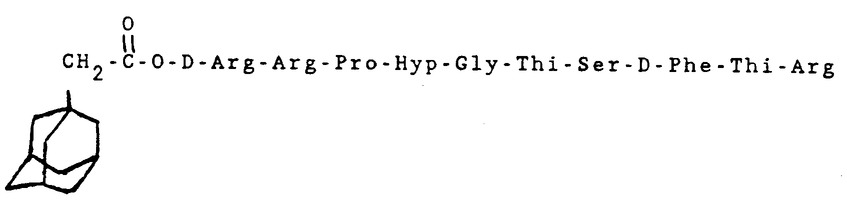
O
||
CH2-C-O-D-Arg-Arg-Pro-Hyp-Gly-Thi-Ser-D-Phe-Thi-Arg
(the test analog) and a non-acylated reference peptide having the structure:
D-Arg-Arg-Pro-Hyp-Gly-Thi-Ser-D-Phe-Thi-Arg, (the model analog) were synthesized by the solid-phase peptide synthesis method by stepwise coupling of BOC-amino acids to the growing peptide chain on a Merrifield resin. Drapeau and Regoli, Methods In Enzymology, 163:263-272 (1988). Adamantaneacetic acid (Sigma Chemical Co., St. Louis, MO) was used in the final coupling step for the test analog. The completion of each coupling reaction was monitored by the Kaiser test. Kaiser et al., Anal. Biochem., 34:595-598 (1970). After each of the protected peptide were assembled, they were cleaved from the support with simultaneous side-chain deprotection by acidolysis in anhydrous hydrogen fluoride (HF) at 0°C in the presence of 15% of anisole. HF was evaporated the mixture was washed with anhydrous diethyl ether and extracted several times with 15% acetic acid. Combined extracts were lyophilized to yield the crude analog. This material was desalted by gel filtration on Sephadex G-15 (50% acetic acid) and purified twice on Sephadex LH-20 (30% acetic acid). The purity and identity of the peptides were ascertained by thin-layer chromatography in two different solvent systems butan-1-ol-acetic acid-water, 4:1:5(v/v) upper phase, and ethyl acetate-pyridine-acetic acid-water, 5:5:1:3 (v/v); and by amino acid analysis.

### EXAMPLE 2

### Bioassay for Antagonistic Activity of Bradykinin Analog

An adamantane acetic acid-modified bradykinin analog was synthesized by the solid-phase method described in Example 1. The analog designed by Stewart et al. was also synthesized and used as a control. The control (or model) and test analogs had the following structure:
Model: D-Arg-Arg-Pro-Hyp-Gly-Thi-Ser-D-Phe-Thi-Arg
Test: 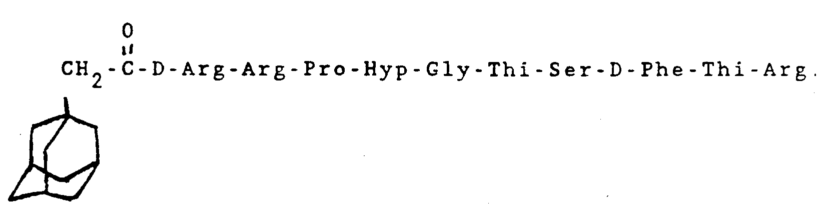
0
||
CH2-C-D-Arg-Arg-Pro-Hyp-Gly-Thi-Ser-D-Phe-Thi-Arg.
The antagonistic potency of the analogs was assessed by their ability to inhibit the vasodepressor response of exogenous bradykinin in awake rats.

Intact male Wistar rats (Charles River Breeding Laboratories, Wilmington, MA, U.S.A.) weighing 225-250 g were used and maintained on a regular Purina Rat Chow diet and tap water in a room at constant temperature (23 ± 1°C) with 12 hour dark/12 hour light cycles. One day before the experiment, the right carotid and the iliac artery in the rats were catheterized with polyethylene tubing (PE 50) under light ether anesthesia. A "Y" type connection was connected to the carotid artery catheter with two ends: one for injecting bradykinin and the other for infusing the bradykinin analogs. All of the catheters were externalized subcutaneously at the back of the neck. On the day of the experiment, the rats were maintained conscious and unrestrained in plastic cages. Arterial pressure (AP), mean arterial pressure (MAP) and heart rate (HR) were monitored through a Gould-Statham P23 ID pressure transducer (Gould, Cleveland, OH) connected to the iliac catheter and recorded on a Gould 2200S paper chart recorder. A half-hour period was allowed before the actual initiation of the experiment. A 1mg/kg amount of angiotensin converting enzyme inhibitor, enalapril (MK-421), was injected into the iliac catheter. Thirty to sixty minutes later, when a stable blood pressure was obtained, two doses of bradykinin acetate salt (Sigma Chemical Co.) of, 125 ng and 250 ng, dissolved in 5% dextrose to a concentration of 2.5 µg/ml, were injected randomly every 4 to 5 minutes into one branch of the carotid catheter. Each dose was repeated two to three times until the vasodepressor responses to exogenous bradykinin were stable. Two average values of the vasodepressor responses to these two doses of bradykinin were taken as the control response. The test and model analogs were infused beginning from a low dose (0.5 µg/min) with a volume no more than 0.2 ml/min (dissolved in 5% dextrose solution), via another branch of the carotid catheter. During each dose of the infusion of the analog, 250 ng of bradykinin was injected into the carotid artery, repeating two to three times until the vasodepressor responses were stable. The average value was taken as the response to 250 ng of bradykinin at the given dose of infusion of the analog. The dose of the analog infusion was increased and the same procedure was repeated until the vasodepressor response to 250 ng of exogenous bradykinin decreased to less than 10% of the control response at the 5th minute, the 10th minute, and then every 10 minutes. The infusion was then discontinued and 250 ng of bradykinin was injected until the vasodepressor response to exogenous bradykinin returned to over 90% of the control level.

The potency of the analog was expressed by the effective dose, ED₂₂ (in low dose) and ED₉₀ (in high dose), which represents the doses of each analog infused (µg/min) which inhibited 22% and 90%, respectively, of the vasodepressor response to its agonist (230 ng of bradykinin).

In another group of rats without pretreatment of enalapril, blood samples (1 ml) were collected from the iliac artery after 5 minute infusion of the analog for measuring plasma catecholamines. Blood samples were centrifuged at 2,500 rpm for 20 minutes at 4°C; the plasma was separated and stored at -80°C until assayed by high-performance liquid chromatography with electrochemical detection.

The effectiveness of each analog was tested by regression analysis of the data obtained in each rat, using a logarithmic transformation of the linear portion of each dose-response curve to calculate the ED₂₂ and ED₉₀ values. The comparison between the two analogs at the same dose was determined using the Students Nonpaired T Test. Differences were considered to be statistically significant if p< 0.05.

### Results

Intracarotid injection of 250 ng and 125 ng of bradykinin decreased MAP by 31.97 ± 1.26 and 24.98 ± 1.29 mmHg (n=14) respectively, in conscious rats pretreated with enalapril (1 mg/kg) without infusion of an analog. The vasodepressor response to 125 ng of bradykinin was 22.21 ± 3.71% (n=14) lower than that to 250 ng of bradykinin. Results are reported as means ± standard error (SE).

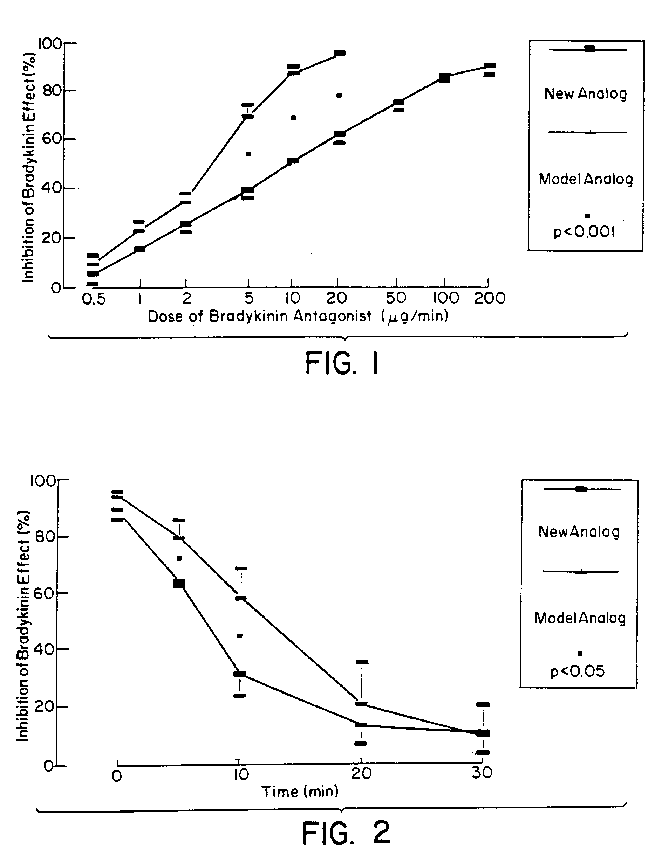

The dose-response curve of the model and test bradykinin analogs is showed in Figure 1. For each given dose, the potency of the new analog was higher than that of the model one. The difference was statistically significant when the doses of the analogs were over 5 µg/min (p<0.001). The ED₂₂ of the new analog was only half that of the model peptide, while the ED₉₀ was more than ten times lower than that of the model one, as shown in Table 1.

**Table 1**

| Effective Dose of Bradykinin Antagonist | | |
|---|---|---|
| | Model Analog | Test Analog |
| Number of the rats | 7 | 7 |
| Weight (g) | 245.14±10.23 | 234.14±6.34 |
| r (regression coefficient) | 0.9892±0.0017 | 0.9826±0.0035 |
| ED₂₂ (µg/min) | 1.49±0.29 | 0.84±0.09 * |
| ED₉₀ (µg/min) | 142.50±26.44 | 13.94±1.69 ** |

| | | |
|---|---|---|
| *: p<0.05, | | |
| **: p<0.001. | | |

ED₂₂ _{and} ED₉₀ represent the doses of the analog infused (µg/min) which inhibit 22% and 90%, respectively, of the vasodepressor response to its agonist (250 ng of BK) in the absence of the antagonist. The model analog was previously designed by Vavrek and Stewart. Vavrek and Stewart, Peptides, 6:161-164 (1985). This analog was synthesized and assayed to obtain the reference data for comparison with the test analog. Although the antagonistic effect of both analogs decreased to less than 10% at the 30th minute after the end of the analog infusion, the effect of the new analog was still significantly higher than of the model one at the 5th and 10 minute after discontinuation shown in Figure 2. No agonistic vasodepressor effect was found when the dose of the new analog was increased to 50 µg/min which as 200-400 times larger than the BK administered.

The pA₂ is the most commonly used parameter to quantitatively express the potency of an antagonist. Schild, Brit. J. Pharmacol., 2:189-206 (1947). The ED value should be the dose of the antagonist that reduces the vasodepressor response to two units of agonist (250 ng of BK) to equal the response to one unit of agonist (125 ng of bradykinin) in the absence of antagonist. In the above bioassay, 125 ng of bradykinin decreased MAP by 24.98±1.29 mmHg, which is 22.21±3.71% lower than that induced by 250 ng of bradykinin (31.97±1.26 mmHg). ED₂₂ was used as the effective dose to express the potency of the bradykinin antagonist during low dose. The pA₂ value represents the negative logarithm of the molar concentration of the effective dose, i.e., the negative logarithm of the ED value divided by the estimated volume of distribution (67ml/kg). Manning et al., J. Med. Chem., 25:45-50 (1982). The control analog gave the anti-vasodepressor potency of the model analog a pA₂ value of 7.17±0.11 compared to 7.42 ± 0.07 for the test analog.

The dose-response curve (Figure 1) illustrates the difference of the antagonistic potency between two analogs in low doses: (below 5 µg/min) the dose response was not as large as that in high doses (over 5 µg/min). The ED₂₂ value of the new analog is about half that of the model one. In order to compare the antagonistic potency between two analogs in higher doses, ED₉₀ was used as a parameter to quantitatively express the dose of the antagonist which inhibits 90% of the vasodepressor response to its agonist (250 ng of bradykinin). Comparison of the ED₉₀ values between the two analogs shows that the new analog was over ten times stronger than the control one which was considered to be the most potent antagonist in a series of bradykinin analogs tested both in vitro and in vivo without any intrinsic effect on systemic blood pressure.

Despite their vascular antagonistic properties, they could also act as an agonist to bradykinin receptors in other tissues (e.g., to stimulate the bradykinin receptor in the adrenal glands to release catecholamine). Some of the bradykinin antagonists were found to have this effect. Mulinari et al., Hypertension, 11:754-757 (1988); Mulinari et al., Hypertension, 13:960-963 (1989). The new bradykinin analogs did not increase plasma catecholamines at a dose of 10 and 50 µg/min, which are the doses that could inhibit more than 90% of the vasodepressor response to the 250 ng of exogenous bradykinin.

In another group of rats, plasma catecholamine level was measured. There was no significant increase in plasma catecholamines when the dose of the new analog went up to 50 µg/min, as shown in Table 2.

**Table 2**

| Plasma Catecholamines | | |
|---|---|---|
| | (pg/ml) Epinephrine | Norepinephrine |
| 5% dextrose (0.1 ml/min, n=6) | 298± 43 | 357±51 |
| BKA (10 µg/min, n=3) | 675±338 | 222±35 |
| BKA (50 µg/min, n=2) | 328±173 | 163±20 |
| BKA = Bradykinin analog | | |

### Equivalents

Those skilled in the art will recognize or be able to ascertain, using no more than routine experimentation, many equivalents to the specific embodiments of the invention described herein. Such equivalents are intended to be encompassed within the scope of the following claims.

## Claims

1. An acylated bradykinin analog selected from: 
O
‖
X-C-Arg-Pro-Hyp-Gly-Thi-Ser-D-Phe-Thi-Arg,

O
‖
X-C-D-Arg-Arg-Hyp-Hyp-Gly-Thi-Ser-D-Phe-Thi-Arg,

O
‖
X-C-Lys-Lys-Arg-Pro-Pro-Gly-Thi-Ser-D-Phe-Thi-Arg and

O
‖
X-C-D-Arg-Arg-Pro-Hyp-Gly-Thi-Ser-D-Phe-Thi-Arg,
 wherein X is an organic substituent selected from the group consisting of: saturated cyclic compounds, unsaturated cyclic compounds, cyclic aromatic compounds, saturated heterocyclic compounds, unsaturated heterocyclic compounds and aromatic heterocyclic compounds.

2. An acylated bradykinin analog of claim 1 having the formula:

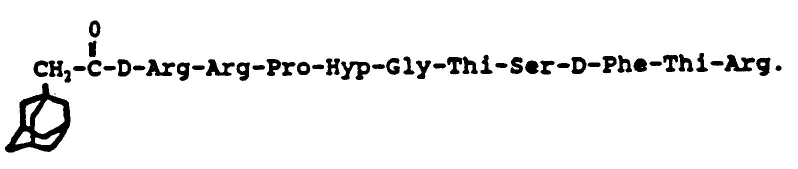
O
‖
CH₂-C-D-Arg-Arg-Pro-Hyp-Gly-Thi-Ser-D-Phe-Thi-Arg.

3. An acylated bradykinin analog of Claim 1 or Claim 2 which is a bradykinin antagonist.

4. Use of an acylated bradykinin analog according to any one of the preceding claims for the manufacture of a medicament for antagonizing the effects of bradykinin in an individual.

5. Use of an acylated bradykinin analog for the manufacture of a medicament to reduce or inhibit the vasodepressor response in an individual, the acylated bradykinin analog having the following formula: 
O
‖
X-C-D-Arg-Arg-Pro-Hyp-Gly-Thi-Ser-D-Phe-Thi-Arg,
 wherein X is an organic substituent selected from the group consisting of: saturated cyclic compounds, unsaturated cyclic compounds, cyclic aromatic compounds, saturated heterocyclic compounds, unsaturated heterocyclic compounds and aromatic heterocyclic compounds.

6. Use according to Claim 5 wherein the amount of the acylated bradykinin analog used is from about 3 to about 15 mg per dose.

7. Use off an acylated bradykinin analog for the manufacture of a medicament for reducing, ameliorating or eliminating a bradykinin-mediated disorder in an individual, the acylated bradykinin analog having the following general formula: 
  O
  ‖
X-C-D-Arg-Arg-Pro-Hyp-Gly-Thi-Ser-D-Phe-Thi-Arg,
 wherein X is an organic substituent selected from the group consisting of: saturated cyclic compounds, unsaturated cyclic compounds, cyclic aromatic compounds, saturated heterocyclic compounds, unsaturated heterocyclic compounds and aromatic heterocyclic compounds.

8. Use according to Claim 7 wherein the amount of the acylated bradykinin analog used is from about 3 to about 15 mg per dose.

9. Use according to Claim 8 wherein the bradykinin-mediated disorder is selected from the group consisting of: angina, sympathetic discharge, cardiac arrhythmia, diabetic neuropathy, ischemic heart disease, burn pain, bronchial asthma, allergic rhinitis, toxic shock, circulatory collapse, oro-facial pain, trigeminal neuralgia, migraine and inflammatory bowel disease.

10. A method of producing the acylated bradykinin analog of any one of claims 1-3 comprising the step of acylating the N-terminal amino group of the bradykinin peptide analogs with a carboxylic acid, e.g. adamantaneacetic acid.

11. An acylated bradykinin analog according to any one of claims 1-3, for use in therapy.

## Patentansprüche

1. Ein acyliertes Bradykinin-Analog ausgewählt von 
O
||
X-C-Arg-Pro-Hyp-Gly-Thi-Ser-D-Phe-Thi-Arg

O
||
X-C-D-Arg-Arg-Hyp-Hyp-Gly-Thi-Ser-D-Phe-Thi-Arg

O
||
X-C-Lys-Lys-Arg-Pro-Pro-Gly-Thi-Ser-D-Phe-Thi-Arg und

O
||
X-C-D-Arg-Arg-Pro-Hyp-Gly-Thi-Ser-D-Phe-Thi-Arg ,
 worin X ein organischer Substituent ist, ausgewählt aus der Gruppe bestehend aus: gesättigten cyclischen Verbindungen, ungesättigten cyclischen Verbindungen, cyclischen aromatischen Verbindungen, gesättigten heterocyclischen Verbindungen, ungesättigten heterocyclischen Verbindungen und aromatischen heterocyclischen Verbindungen.

2. Ein acyliertes Bradykinin-Analog gemäß Patentanspruch 1 mit der Formel:

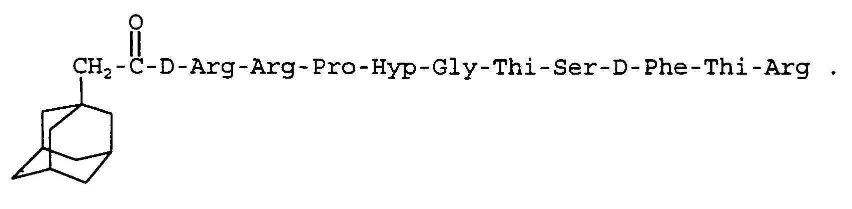
O
‖
CH2-C-D-Arg-Arg-Pro-Hyp-Gly-Thi-Ser-D-Phe-Thi-Arg .

3. Ein acyliertes Bradykinin-Analog gemäß Patentanspruch 1 oder Patentanspruch 2, das ein Bradykinin-Antagonist ist.

4. Verwendung des acylierten Bradikinin-Analogs gemäß einem der vorhergehenden Patentansprüche zur Herstellung eines Medikaments, um den Auswirkungen von Bradykinin in einem Individuum entgegenzuwirken.

5. Verwendung eines acylierten Bradykinin-Analogs zur Herstellung eines Medikaments zur Reduzierung oder Inhibierung der Vasodepressor-Wirkung in einem Individuum, wobei das acylierte Bradykinin-Analog die folgende Formel hat: 
O
||
X-C-D-Arg-Arg-Pro-Hyp-Gly-Thi-Ser-D-Phe-Thi-Arg,
 worin X ein organischer Substituent ist, ausgewählt aus der Gruppe bestehend aus: gesättigten cyclischen Verbindungen, ungesättigten cyclischen Verbindungen, cyclischen aromatischen Verbindungen, gesättigten heterocyclischen Verbindungen, ungesättigten heterocyclischen Verbindungen und aromatischen heterocyclischen Verbindungen.

6. Verwendung gemäß Patentanspruch 5, wobei die eingesetzte Menge des acylierten Bradykinin-Analogs etwa 3 bis 15 mg per Dosis ausmacht.

7. Verwendung des acylierten Bradykinin-Analogs zur Herstellung eines Medikaments zur Reduzierung, Verbesserung oder Eliminierung einer Bradykinin - verursachten Funktionsstörung in einem Individuum, wobei das acylierte Bradykinin-Analog die folgende allgemeine Formel hat: 
O
||
X-C-D-Arg-Arg-Pro-Hyp-Gly-Thi-Ser-D-Phe-Thi-Arg,
 worin X ein organischer Substituent ist ausgewählt aus der Gruppe bestehend aus: gesättigten cyclischen Verbindungen, ungesättigten cyclischen Verbindungen, cyclischen aromatischen Verbindungen, gesättigten heterocyclischen Verbindungen, ungesättigten heterocyclischen Verbindungen und aromatischen heterocyclischen Verbindungen.

8. Verwendung gemäß Patentanspruch 7, wobei die verwendete Menge des acylierten Bradykinin-Analogs etwa 3 bis 15 mg per Dosis ausmacht.

9. Verwendung gemäß Patentanspruch 8, wobei die Bradykinin-verursachte Funktionsstörung ausgewählt ist aus der Gruppe bestehend aus: Angina, Sympathicus-bedingte Freisetzung (sympathetic discharge), Herzarrhythmie, diabetische Neuropathie, Ischemische Herzkrankheit, durch Verbrennungen verursachte Schmerzen, Bronchialasthma, allergische Rhinitis, toxischer Schock, Kreislaufkollaps, orofaciale Schmerzen, Trigeminus-Neuralgie, Migräne und entzündliche Darmkrankheiten.

10. Ein Verfahren zur Herstellung des acylierten Bradykinin-Analogs entsprechend einem der Patentansprüche 1 bis 3 bestehend aus oder enthaltend den Schritt der Acylierung der N-terminalen Amino-Gruppe des Bradykinin-Peptid-Analogs mit Carbonsäure, beispielsweise Adamantanessigsäure.

11. Ein acyliertes Bradikinin-Analog gemäß einem der Patentansprüche 1 bis 3 zur Verwendung in der Therapie.

## Revendications

1. Un analogue acylé de la bradykinine, sélectionné parmi: 
O
||
X-C-Arg-Pro-Hyp-Gly-Thi-Ser-D-Phe-Thi-Arg,
O
||
X-C-D-Arg-Arg-Hyp-Hyp-Gly-Thi-Ser-D-Phe-Thi-Arg,
O
||
X-C-Lys-Lys-Arg-Pro-Pro-Gly-Thi-Ser-D-Phe-Thi-Arg et
O
||
X-C-D-Arg-Arg-Pro-Hyp-Gly-Thi-Ser-D-Phe-Thi-Arg
 dans lesquels X est un substituant organique sélectionné dans le groupe consistant en: composés cycliques saturés, composés cycliques insaturés, composés aromatiques cycliques, composés hétérocycliques saturés, composés hétérocycliques insaturés et composés hétérocycliques aromatiques.

2. Un analogue acylé de la bradykinine, de la revendication 1, ayant la formule:

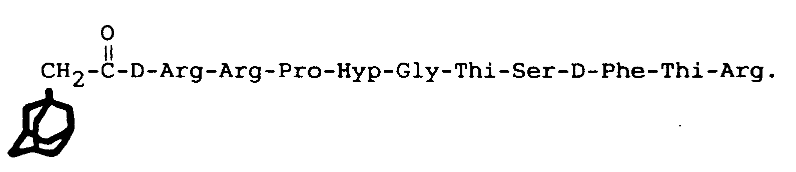
O
||
CH2-C-D-Arg-Arg-Pro-Hyp-Gly-Thi-Ser-D-Phe-Thi-Arg.

3. Un analogue acylé de la bradykinine, de la revendication 1 ou de la revendication 2, qui est un antagoniste de la bradykinine.

4. Utilisation d'un analogue acylé de la bradykinine, selon l'une quelconque des revendications précédentes, pour la fabrication d'un médicament pour s'opposer aux effets de la bradykinine, chez un individu.

5. Utilisation d'un analogue acylé de la bradykinine, pour la fabrication d'un médicament pour réduire ou inhiber la réponse à un vasodépresseur, chez un individu, l'analogue acylé de la bradykinine ayant la formule suivante: 
  O
  ||
X-C-D-Arg-Arg-Pro-Hyp-Gly-Thi-Ser-D-Phe-Thi-Arg,
 dans laquelle X est un substituant organique sélectionné dans le groupe consistant en: composés cycliques saturés, composés cycliques insaturés, composés aromatiques cycliques, composés hétérocycliques saturés, composés hétérocycliques insaturés et composés hétérocycliques aromatiques.

6. Utilisation selon la revendication 5, dans laquelle la quantité d'analogue acylé de la bradykinine utilisée est d'environ 3 à environ 15 mg par dose.

7. Utilisation d'un analogue acylé de la bradykinine pour la fabrication d'un médicament pour réduire, améliorer ou éliminer un trouble dont la bradykinine est le médiateur, chez un individu, l'analogue acylé de la bradykinine ayant la formule générale suivante: 
O
||
X-C-D-Arg-Arg-Pro-Hyp-Gly-Thi-Ser-D-Phe-Thi-Arg,
 dans laquelle X est un substituant organique sélectionné dans le groupe consistant en: composés cycliques saturés, composés cycliques insaturés, composés aromatiques cycliques, composés hétérocycliques saturés, composés hétérocycliques insaturés et composés hétérocycliques aromatiques.

8. Utilisation, selon la revendication 7, dans laquelle la quantité d'analogue acylé de la bradykinine utilisée est d'environ 3 à environ 15 mg par dose.

9. Utilisation selon la revendication 8, dans laquelle le trouble dont la bradykinine est le médiateur, est sélectionné dans le groupe consistant en: angine, décharge symphatique, arythmie cardiaque, névropathie diabétique, maladie de coeur ischémique, douleur causée par une brûlure, asthme bronchique, rhinite allergique, choc toxique, collapsus circulatoire, douleur oro-faciale, névralgie du trijumeau, migraine et maladie inflammatoire des intestins.

10. Une méthode de production de l'analogue acylé de la bradykinine d'une quelconque des revendications 1-3, comprenant l'étape d'acyler le groupe amino N-terminal des analogues peptidiques de la bradykinine, avec un acide carboxylique, par exemple, l'acide adamantane acétique.

11. Un analogue acylé de la bradykinine, selon l'une quelconque des revendications 1-3, pour une utilisation en thérapie.
